# EUROPEAN PATENT SPECIFICATION

(11) **EP 1 821 432 B1**
(45) Date of publication and mention of the grant of the patent: **06.07.2011**
(21) Application number: 05814680.4
(22) Date of filing: 07.12.2005
(51) Int. Cl.: H04B 13/00, A61B 5/00

(54) **INFORMATION TRANSMISSION THROUGH-HUMAN-BODY SYSTEM AND TRANSMITTER/RECEIVER**
INFORMATIONSÜBERTRAGUNG ÜBER EIN MENSCHENKÖRPERSYSTEM UND EMPFÄNGER/SENDER
SYSTEME DE TRANSMISSION D'INFORMATIONS PAR LE CORPS HUMAIN ET EMETTEUR/RECEPTEUR

(30) Priority: 08.12.2004 JP 2004355514
(43) Date of publication of application: 22.08.2007
(73) Proprietor: Seiko Instruments Inc., Chiba-shi, Chiba 261-8507 (JP)
(72) Inventor: TSUJI, Tomoharu, Seiko Instruments Inc., Chiba; 2618507 (JP)
(74) Representative: Cloughley, Peter Andrew
(86) International application number: PCT/JP2005/022423
(87) International publication number: WO 2006/062112

(56) References cited:
- WO-A-00/15931
- DE-A1- 19 902 983
- JP-A- 2002 152 145
- JP-A- 2002 246 987
- JP-A- 2003 318 841
- JP-A- 2004 134 996
- US-A- 5 914 701

## Description

The present invention relates to a via-human-body information transmission system performing an information transmission through a human body.

### [Background Art]

In recent years, there are eagerly proposed communications having utilized the human body as a transmission line (for example, refer to Patent Documents 1 - 3).

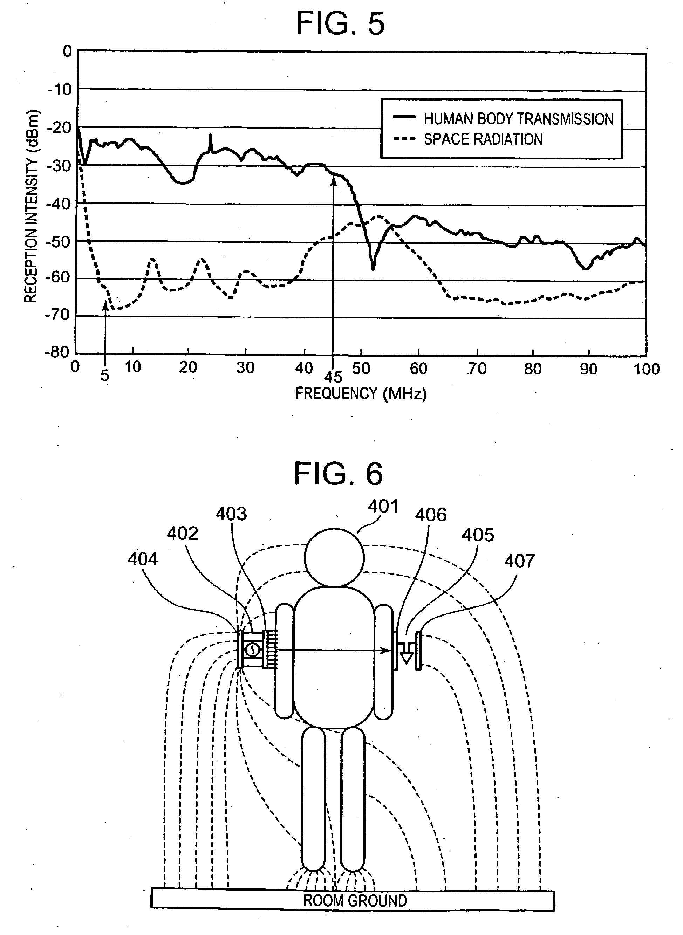

Fig. 6 is a system constitution view of the via-human-body information transmission system having been described in the Patent Document 1. In Fig. 6, a transmitter 402 is worn to one arm of a human body 401, and a receiver 405 is worn to The other arm. One electrode 403 of the transmitter 402 contacts with or is capacitance-connected to the human body 401, and the other electrode 404 is attached while being directed to an outside of the human body 401. Further, one electrode 406 of the receiver 405 contacts with or is capacitance-connected to the human body 401, and the other electrode 407 is attached while being directed to the outside of the human body 401.
In a signal line side, the transmitter 402 is connected to the receiver 405 through the electrode 403, the human body 401 and the electrode 406. Further, in a reference potential line (e.g., ground potential) side, the receiver 405 is connected to the transmitter 402 by such paths by the capacitance connection as shown by broken lines through the electrode 407, a reference potential point (e.g., ground) and the electrode 404.

In the via-human-body information transmission system having been constituted like the above, a signal having been transmitted from the transmitter 402 is transmitted to the receiver 405 through the electrode 403, the human body 401 and the electrode 406. By doing like this, although it is possible to transmit the signal having been outputted from the transmitter 402 to the receiver 405, there are problems that a signal transmission operation is unstable because a length, through which the capacitance connection is utilizes, is long, and that a miniaturization is difficult because it is necessary to increase areas of the electrodes 403, 404, 406, 407.

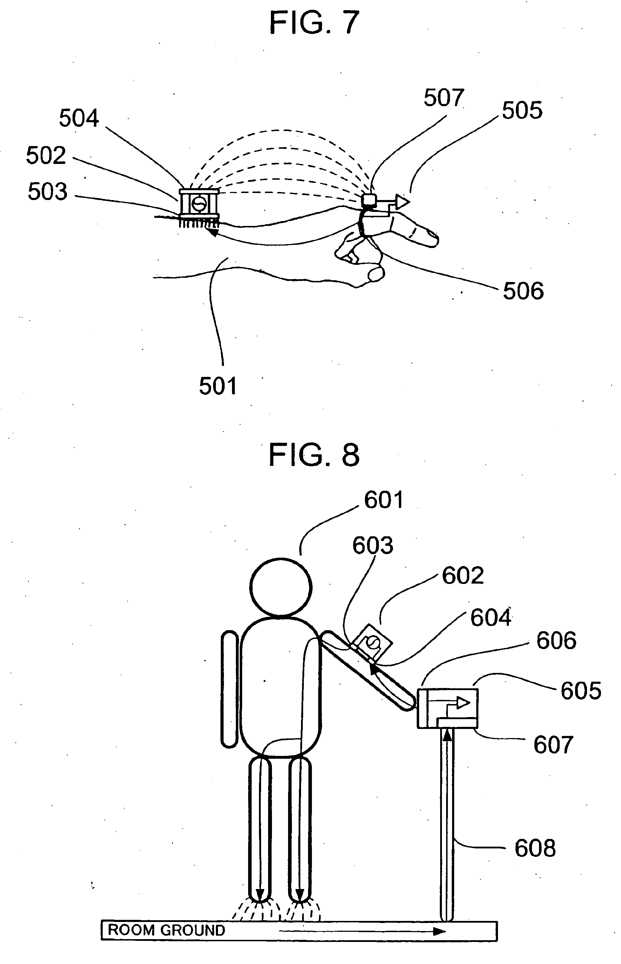

Fig. 7 is a system constitution view of the via-human-body information transmission system having been described in the Patent Document 2. In Fig. 7, a transmitter 502 is attached to an arm of a human body 501, and a receiver 505 is attached to a finger. One electrode 503 of the transmitter 502 contacts with or is capacitance-connected to the human body 501, and the other electrode 504 is attached while being directed to an outside of the human body 501. Further, one electrode 506 of the receiver 505 contacts with or is capacitance-connected to the human body 501, and the other electrode 507 is attached while being directed to the outside of the human body 501.
In the signal line side, the transmitter 502 is electrically connected to the receiver 505 by such paths of the capacitance connection as shown by broken lines through the electrode 504 and the electrode 507. Further, in the reference potential line (e.g., ground potential) side, the receiver 505 is connected to the transmitter 502 through the electrode 506, the human body 501 and the electrode 503.

In the via-human-body information transmission system having been constituted like the above, a signal having been transmitted from the transmitter 502 is transmitted to the receiver 505 by the paths by the capacitance connection through the electrode 504 and the electrode 507. By doing like this, although it is possible to transmit the signal having been outputted from the transmitter 502 to the receiver 505, since it is a constitution in which both of the electrode 504 and the electrode 507 have been attached while being directed to the outside of the human body 501, there is a problem that an intensity of the capacitance connection changes by a person' s motion and thus a signal transmission operation becomes unstable. Further, there is a problem that a distance between the transmitter 502 and the receiver 505 cannot be made long.

Fig. 8 is a system constitution view of the via-human-body information transmission system having been described in the Patent Document 3. In Fig. 8, a first communication equipment 602 is worn to one arm of a human body 601. A second communication equipment 605 is attached to a supporting column 608 of a conductor, which has been connected to the reference potential (e.g., ground potential). Two electrodes 603, 604 of the communication equipment 602 contact with or are capacitance-connected to the human body 601. Further, one electrode 606 of the communication equipment 605 is attached while being directed to a space, and the other electrode 607 is attached to the supporting column 608 so as to be electrically connected thereto.
In the signal line side, the communication equipment 605 is connected to the communication equipment 602 through the electrode 606, the human body 601 and the electrode 604. Further, in a reference potential line (e.g., ground potential) side, the communication equipment 602 is connected to the communication equipment 605 through the electrode 603, the human body 601, paths by the capacitance connection shown by broken lines, the reference potential (ground potential), the supporting column 608 and the electrode 607.

In the via-human-body information transmission system having been constituted like the above, if a fingertip of the human body 601 is contacted with the electrode 606, the communication equipment 605 transmits an information to the communication equipment 602 through the electrode 606, the human body 601 and the electrode 604. Incidentally, at this time, although the two electrodes 603, 604 are attached so as to contact with or be capacitance-connected to the human body 601, between the electrode 603 and the electrode 604, since there is no short circuit and there exists an impedance of an intensity of certain degree, it is possible to transmit a signal having been outputted from the communication equipment 605 to the communication equipment 602.
However, since the system of Fig. 8 is a system whose object is a use as an ID tag, it is constituted so as to be used with only one communication equipment being worn to the human body, and thus there is not considered such a constitution that plural communication equipment are worn to the human body and the communication is performed between the plural communication equipment.

[Patent Document 1] US Patent No. 5914701 Gazette, column 1, line 60-column 6, line 37, Fig. 1 - Fig. 4
[Patent Document 2] Japanese Patent No. 3425347 Gazette, columns [0034] - [0046], Fig. 1 - Fig. 3
[Patent Document 3] JP-A-2001-308803 Gazette, columns [0019] - [0030], Fig.1 - Fig. 6
United States Patent No. 5,914,701 describes a via-human-body information transmission system comprising a first pair of electrodes, which are fed by a signal generator, and a second pair of electrodes, which are connected to a signal detector. An inner electrode of the first pair of electrodes is closely capacitively coupled to one arm of the user, while an inner electrode of the second pair of electrodes is closely capacitively coupled to the user's other arm. An outer-facing electrode of each of the first and second pairs of electrodes is capacitively coupled to the ground of the room, in which the user is located. Hence current flows from the generator to the detector in two paths: a first path directly from one arm of the user to the other arm of the user, and a second path via the room ground.
PCT patent application published as WO 00/15931 discloses a via-human-body information transmission system for use with a vehicle. The transmission system comprises a base station, which is located in the vehicle, and a movable data carrier, which is located outside the vehicle. Each of the base station and the data carrier has a data signal processing circuit. The data signal processing circuit of the data carrier is connected to a first electrode, which is capacitively coupled to the vehicle ground, and to a second electrode, which is capacitively connected to the body of the user. A third electrode is capacitively coupled to another part of the user's body and ohmically coupled to the data signal processing circuit of the base station, while the data signal processing circuit of the base station is also ohmically coupled to vehicle ground via a fourth electrode.

### [Disclosure of the Invention]

### [Problems that the Invention is to Solve]

Problems of the present invention are to constitute, in the via-human-body information transmission system performing the information transmission between plural communication means through the human body, so as to be used while attaching the plural communication means to the human body, and to make such that the information transmission can be more stably performed.
Further, a problem of the present invention is to simplify a constitution in the via-human-body information transmission system.
Further, a problem of the present invention is to provide a transmitter-receiver suitable for constructing the via-human-body information transmission system.

### [Means for Solving the Problems]

According to the present invention, there is provided a via-human-body information transmission system as set forth in claim 1.

Here, there may be constituted such that an organism information of the human body is transmitted between the first communication means and the second communication means, and the first and second electrodes of the first communication means are used both for an electrode detecting the organism information of the human body and for an electrode performing a transmission of the organism information.
Further, there may be constituted such that a frequency of a carrier signal used in the communication between the first communication means and the second communication means is set to a frequency different from a frequency of the organism information of the human body.

Further, there may be constituted such that the frequency of the carrier signal is a frequency within a range of 5 MHz - 45 MHz.
Further, there may be constituted such that the organism information is an information of a heartbeat.
Further, there may be constituted such that the first communication means is attached to the human body by a chest band.

Further, there may be constituted such that the first communication means has a filter through which a signal of a predetermined frequency in a signal having been received by the first electrode passes and which outputs it, a heartbeat detecting section detecting a heartbeat signal from an output signal of the filter, a first processor section calculating a heartbeat number on the basis of the heartbeat signal having been detected in the heartbeat detecting section to thereby output a heartbeat number data signal denoting the heartbeat number, a modulating section modulating and outputting the heartbeat number data signal, and a data transmitting section outputting the heartbeat number data signal having been modulated in the modulating section, and the second communication means has a demodulating section demodulating the heartbeat number data signal having been received by the third electrode, a storage means, a display section, and a second processor section which stores a heartbeat number data having been obtained from the heartbeat number data signal having been demodulated in the demodulating section to the storage means and which display-processes the heartbeat number to the display section.

### [Advantages of the Invention]

According to the via-human-body information transmission system concerned with the present invention, since it becomes possible to shorten a distance through which the capacitance connection is performed, it becomes possible to more stabilize an information transmission operation.
Further, by the fact that the electrode used for the detection of the organism information and the electrode used for the communication are commonly used, it becomes unnecessary to provide a new communication means, so that it becomes possible to inexpensively constitute by a simple constitution.
Further, according to the present invention, it becomes possible to provide a transmitter-receiver suitable for constructing the via-human-body information transmission system.

### [Brief Description of the Drawings]

[Fig. 1] It is a system constitution view of a via-human-body information transmission system concerned with an embodiment of the present invention.
[Fig. 2] It is a detailed block diagram of a transmitter-receiver used in the via-human-body information transmission system concerned with the embodiment of the present invention.
[Fig. 3] It is a detailed block diagram of a receiver used in the via-human-body information transmission system concerned with the embodiment of the present invention.
[Fig. 4] It is a view when measuring a characteristic of the via-human-body information transmission system concerned with the embodiment of the present invention.
[Fig. 5] It is a characteristic diagram showing the characteristic of the via-human-body information transmission system concerned with the embodiment of the present invention.
[Fig. 6] It is a system constitution view of a conventional via-human-body information transmission system.
[Fig. 7] It is a system constitution view of a conventional via-human-body information transmission system.
[Fig. 8] It is a system constitution view of a conventional via-human-body information transmission system.

### [Description of Reference Numerals]

- 101: human body
- 102: transmitter-receiver as first communication means
- 103: transmitter-receiver main body
- 104: first electrode
- 105: second electrode
- 106: receiver as second communication means
- 107: receiver main body
- 108: third electrode
- 109: fourth electrode
- 110: chest belt
- 111: measuring point
- 201, 301: amplifying section
- 202: filter
- 203: heartbeat detecting section
- 204: first processor section
- 205: modulating section
- 206: data transmitting section
- 302: demodulating section
- 303: second processor section
- 304: memory as storage means
- 305: display section as display means

### [Best Mode for Carrying Out the Invention]

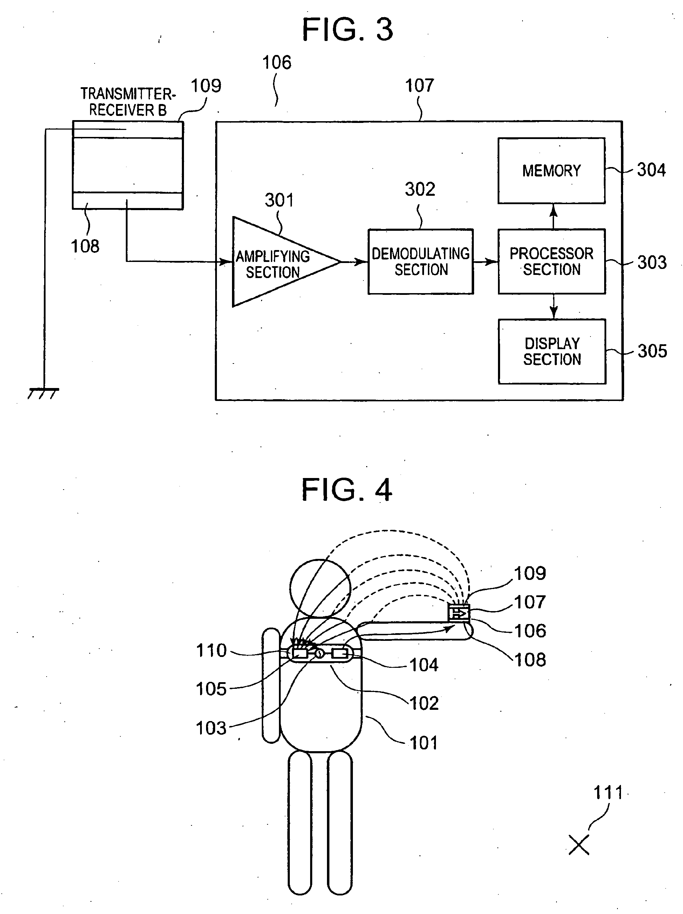

Hereunder, there are explained about a via-human-body information transmission system and a transmitter-receiver, which are concerned with an embodiment of the present invention.

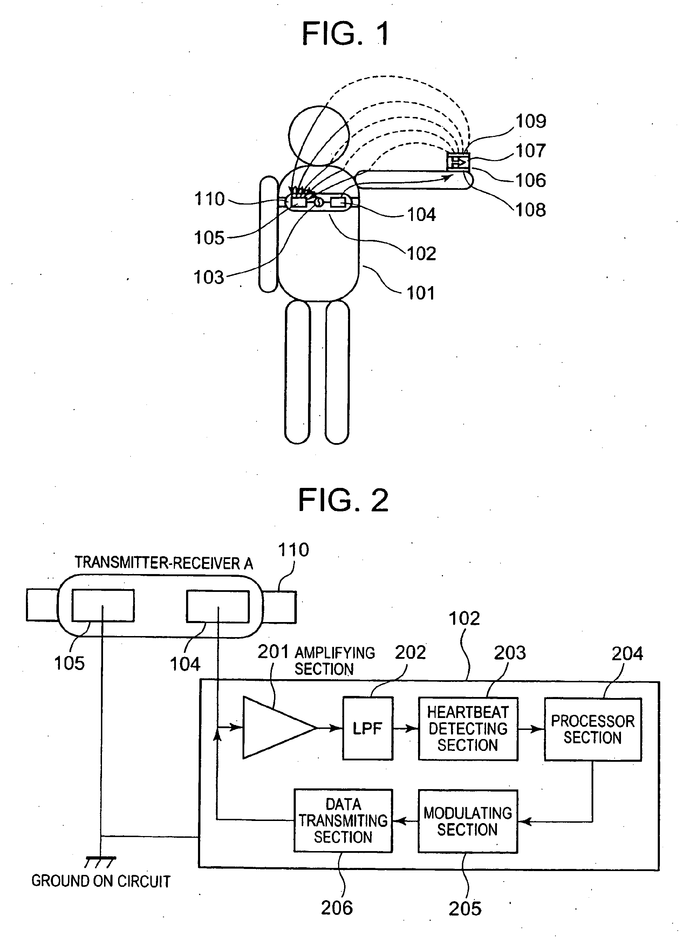

Fig. 1 is a system constitution view of the via-human-body information transmission system concerned with the embodiment of the present invention, and shows an example of an ictometer system as the via-human-body information transmission system.
Further, Fig. 2 is a detailed block diagram of a transmitter-receiver 102, and Fig. 3 is a detailed block diagram of a receiver 106. Incidentally, in Fig. 1 - Fig. 3, the same reference numeral is applied to the same portion.

In Fig. 1 - Fig. 3, the transmitter-receiver 102 as a first communication means and the receiver 106 as a second communication means are both used while being attached to a human body 101. The transmitter-receiver 102 is attached to a chest part of the human body 101 by a chest band 110. The receiver 106 is attached to one arm (e.g., wrist of a left arm) of the human body 101.
The transmitter-receiver 102 has a transmitter-receiver main body 103, a first electrode 104 contacting with or capacitance-connected to the human body 101, and a second electrode 105 contacting with or capacitance-connected to the human body 101. Further, the receiver 106 has a receiver main body 107, a third electrode 108 contacting with or capacitance-connected to the human body 101, and a fourth electrode 109 having been disposed while being directed to an outside (opposite side to the human body 101) of the human body 101.

The first electrode 104 and the third electrode 108 are electrically connected through the human body 101 (mainly, the chest part and the left arm that is the arm to which the receiver 106 has been attached), and the second electrode 105 and the fourth electrode 109 are electrically connected by a capacitance connection as shown by broken lines. By this, there is constituted such that a communication is possible between the transmitter-receiver 102 and the receiver 106.
That is, in the signal line side, the transmitter-receiver 102 is connected to the receiver 106 through the electrode 104, the human body 101 and the electrode 108. Further, in the reference potential line (e.g., ground potential) side, the receiver 106 is connected to the transmitter-receiver 102 by the paths by such a capacitance connection as shown by the broken lines through the electrode 109, the human body 101 and the electrode 105.
Even in any case of a case where the electrode 104 and the electrode 105 have been attached so as to contact with the human body, and a case where they have been attached so as to be capacitance-connected to the human body, between the electrode 104 and the electrode 105, since there is no short circuit and there exists the impedance, a signal transmission between the transmitter-receiver 102 and the receiver 106 becomes possible.

The transmitter-receiver main body 103 has an amplifying section 201 amplifying and outputting a signal having been received by the first electrode 104, a filter (low-pass filter in the present embodiment) 202 through which a signal of predetermined frequency in an output signal of the amplifying section 201 passes, a heartbeat detecting section 203 detecting a heartbeat signal from an output signal of the filter 202, a first processor section 204 calculating a heartbeat number on the basis of the heartbeat signal having been detected in the heartbeat detecting section 203 to thereby output a heartbeat number data signal denoting the heartbeat number, a modulating section 205 modulating and outputting the heartbeat number data signal, and a data transmitting section 206 outputting the heartbeat number data signal having been modulated in the modulating section 205 from the electrode 104.

The filter 202 is a filter for removing a noise signal other than the heartbeat signal. Further, a frequency of a carrier signal that the modulating section 205 uses for modulating the heartbeat number data signal, i.e., a frequency of a carrier signal used in a communication between the transmitter-receiver 102 and the receiver 106, is set so as to differ from a frequency of the heartbeat signal. By this, there is constituted such that a signal communicated between the transmitter-receiver 102 and the receiver 106 and the heartbeat signal do not interfere. Incidentally, about a method of determining the frequency of the carrier signal, there is mentioned later.

The receiver main body 107 has an amplifying section 301 amplifying and outputting the heartbeat number data signal having been received by the third electrode 108, a demodulating section 302 demodulating an output signal of the amplifying section 301, a memory 304 as a storage means, a display section 305 as a display means, and a second processor section 303 which obtains a heartbeat number from the heartbeat number data signal having been demodulated by the demodulating section 302 to thereby store the heartbeat number to the memory 304, and which display-processes the heartbeat number to the display section 305.

In an ictometer system having been constituted like the above, in a case where the transmitter-receiver 102 is in a detecting mode, the amplifying section 201 amplifies and outputs the heartbeat signal having been received (detected) by the electrode 104. The filter 202 causes the signal of predetermined frequency in the output signal of the amplifying section 201 to pass and outputs it. The heartbeat detecting section 203 detects and outputs the heartbeat signal from the output signal of the filter 202. The processor section 204 calculates the heartbeat number on the basis of the heartbeat signal having been detected in the heartbeat detecting section 203.

Next, if the transmitter-receiver 102 becomes a transmitting mode, the processor section 204 outputs the heartbeat number data signal denoting the heartbeat number having been calculated on the basis of the heartbeat signal having been detected in the heartbeat detecting section 203 to the modulating section 205. The modulating section 205 modulates the heartbeat number data signal from the processor section 204 by the carrier signal of predetermined frequency, and outputs it. The data transmitting section 206 outputs the heartbeat number data signal having been modulated in the modulating section 205 from the electrode 104.

The heartbeat number data signal having been outputted from the electrode 104 is received by the third electrode 108 of the receiver 106 while passing the human body 101 (mainly, the chest part and the left arm).
The receiver main body 107 amplifies the heartbeat number data signal having been received by the electrode 108 in the amplifying section 301, and outputs it. The demodulating section 302 demodulates and outputs the heartbeat number data signal having been outputted from the amplifying section 301. The second processor section 303 obtains the heartbeat number from the heartbeat number data signal having been outputted from the demodulating section 302 to thereby store in order the heartbeat number to the memory 304 in order by which the heartbeat number data signal has been received, and displays the heartbeat number to the display part 305.

As having been mentioned above, the via-human-body information transmission system concerned with the embodiment of the present invention possesses the transmitter-receiver 102 as a first communication means, which has the two electrodes 104, 105 contacting with or capacitance-connected to the human body 101, and which is used while being attached to the human body 101, and the receiver 106 as a second communication means, which has the one electrode 108 contacting with or capacitance-connected to the human body 101 and the one electrode 109 having been disposed while being directed to the outside of the human body 101, and which is used while being attached to the human body 101.
Accordingly, since it becomes possible to constitute the distance, by which the capacitance connection is performed, shortly in some extent, there becomes such that a communication operation between the transmitter-receiver 102 and the receiver 106 is more stabilized.

Further, since the transmitter-receiver 102 and the receiver 106 are attached respectively to the chest and the arm and a signal path via the human body 101 is long, it becomes possible to reduce an influence by a person's posture and the like in comparison with a case where the transmission path by the capacitance connection is long.
Further, since the two electrodes 104, 105 of the transmitter-receiver 102 are used both for the electrode performing the transmission/reception of the signal and for the electrode detecting an organism information of the human body 101, the communication of the signal and the detection of the organism information become possible by a simple constitution. Further, it becomes unnecessary to newly provide a communicating circuit such as RF circuit, so that a great reduction in cost becomes possible.

Further, since there is constituted such that a carrier frequency of the transmitter-receiver 102 and a frequency of the organism information (e.g., the heartbeat signal) are different, there is brought about such an advantage that it becomes possible to suppress the interference between a transmission signal and an organism information signal.
Further, there is constituted such that the transmitter-receiver 102 is attached to the chest of the human body 101 and the receiver 106 is attached to the arm of the human body 101, so that such a constitution as to enlarge the distance between the transmitter-receiver 102 and the receiver 106 is possible.
Further, according to the transmitter-receiver 102 concerned with the embodiment of the present invention, since the above via-human-body information transmission system can be constructed and especially the electrodes are used both for the communication and for the organism information detection, there is bought about an advantage that it becomes possible to simplify the constitution.

Next, there is explained about the method of determining the carrier signal frequency of the transmitter-receiver 102.
Fig. 4 is an explanatory view when determining the carrier frequency of the transmitter-receiver 102 in the above embodiment. In Fig. 4, the same reference numeral is applied to the same portion as Fig. 1, and an antenna (not shown in the drawing) for measurement is disposed at a measuring point 111 having been separated from the transmitter-receiver 102 by a predetermined distance.
Further, Fig. 5 is a characteristic diagram showing a frequency characteristic of the embodiment, wherein a solid line denotes a reception intensity of a signal having been received from the transmitter-receiver 102 by the electrode 108 of the receiver 106, and a broken line denotes a reception intensity of a signal having been received by the antenna at the measuring point 111.

In Fig. 4 and Fig. 5, the receiver 106 receives a signal from the transmitter-receiver 102 mainly via the human body. On the other hand, at the measuring point 111, a signal having propagated in the air from the transmitter-receiver 102 is received by the antenna and measured. From viewpoints of well performing the transmission of the signal and reducing an unnecessary radiation, it is desirable that an intensity of the signal that the receiver 106 has received from the transmitter-receiver 102 is large, and the intensity at the measuring point 111 is rather small.

In the present embodiment, from such viewpoints, there is adapted such that there is used the carrier signal within a range from 5 MHZ that is a frequency at which the intensity of the signal that the receiver 106 has received from the transmitter-receiver 102 does not decrease and the intensity of the signal having been measured at the measuring point 111 greatly decreases to 45 MHz that is a frequency at which the intensity of the signal that the receiver 106 has received from the transmitter-receiver 102 starts to decrease and the intensity of the signal having been measured at the measuring point 111 does not so increase.
Like this, by making the frequency of the carrier signal that the modulating section 205 of the transmission/reception part 102 uses for modulating the heartbeat number data signal into the frequency within the range of 5 MHz - 45 MHz, a good signal transmission becomes possible because the receiver 106 can receive the signal of a constant intensity from the transmitter-receiver 102, and it becomes possible to suppress low the unnecessary radiation.

Incidentally, in the above embodiment, a switching of the detecting mode and the transmitting mode in the transmitter-receiver 102 may be made so as to be automatically performed for every predetermined time, and further such various modifications are possible as to constitute such that a start or the switching of the detecting mode and the transmitting mode is operated by providing an operation part in the transmitter-receiver 102 and operating the operation part by a user.
Further, although the transmitter-receiver 102 has been given as an example of the first communication means, and the reception part 106 has been given as an example of the second communication means, various modifications are possible if it is a constitution capable of performing the communication between the first and second communication means such as to make the reception part 106 into a data logger exclusively for collecting the data, to make both of the first and second communication means into the transmitter-receiver, or to constitute so as to make the first and second communication means respectively into the receiver and the transmitter-receiver.

### [Industrial Applicability]

As the via-human-body information transmission system, there can be applied not only to the ictometer but also to such various systems as to perform the communication between the plural communication means having been worn to the human body, besides an organism information measurement system such as pulsimeter and pedometer.

## Claims

1. A via-human-body information transmission system for performing an information transmission between plural communication means through a human body (101), comprising:
a first communication means (102) which has a first electrode (104) disposed such as to face toward the human body (101) and being directly in contact with or capacitively connected to the human body, and a second electrode (105), and which is used while being attached to the human body, and
a second communication means (106), which has a third electrode (108) disposed such as to face toward the human body (101) and being directly in contact with or capacitively connected to the human body and a fourth electrode (109) disposed such as to face away from the human body, and which is used while being attached to the human body;
wherein the information transmission system is adapted to perform a communication between the first and second communication means (102, 106) by an electrical connection between the first electrode (104) and the third electrode (108) through the human body, and by a capacitive connection between the second electrode (105) and the fourth electrode (109);
**characterised in that**:
the second electrode (105) is disposed such as to face toward the human body (101) and is directly in contact with or is capacitively connected to the human body;
the third and fourth electrodes (108, 109) are separated from each other by means of a main body (107), the third and fourth electrodes (108, 109) being disposed at opposite ends of the main body (107); and
the capacitive connection between the second and fourth electrodes (105, 109) is by way of the human body (101).

2. A via-human-body information transmission system according to claim 1, **characterised in that** the information transmission system is adapted to transmit an organism information of the human body (101) between the first communication means (102) and the second communication means (106), and to use the first and second electrodes (104, 105) of the first communication means both for an electrode detecting the organism information of the human body and for an electrode performing a transmission of the organism information.

3. A via-human-body information transmission system according to claim 1 or 2, **characterised in that** the information transmission system is adapted to set a frequency of a carrier signal used in the communication between the first communication means (102) and the second communication means (106) to a frequency different from a frequency of the organism information of the human body (101).

4. A via-human-body information transmission system according to claim 3, **characterised in that** the frequency of the carrier signal is a frequency within a range of 5 MHz - 45 MHz.

5. A via-human-body information transmission system according to any one of claims 2 to 4, **characterised in that** the organism information is an information of a heartbeat.

6. A via-human-body information transmission system according to any one of claims 1 to 5, **characterised in that** the first communication means (102) is attached to the human body (101) by a chest band (110).

7. A via-human-body information transmission system according to any one of claims 1 to 6, **characterised in that** the first communication means (102) has a filter (202) adapted to pass and to output a signal of a predetermined frequency in a signal having been received by the first electrode (104), a heartbeat detecting section (203) adapted to detect a heartbeat signal from an output signal of the filter, a first processor section (204) adapted to calculate a heartbeat number on the basis of the heartbeat signal having been detected in the heartbeat detecting section to thereby output a heartbeat number data signal denoting the heartbeat number, a modulating section (205) adapted to modulate and output the heartbeat number data signal, and a data transmitting section (206) adapted to output the heartbeat number data signal having been modulated in the modulating section, and
the second communication means (106) has a demodulating section (302) adapted to demodulate the heartbeat number data signal having been received by the third electrode (108), a storage means (304), a display section (305), and a second processor section (303) adapted to store in the storage means (304) a heartbeat number data having been obtained from the heartbeat number data signal having been demodulated in the demodulating section, and to display-process the heartbeat number to the display section (305).

## Patentansprüche

1. Informationsübertragungssystem über den menschlichen Körper zur Durchführung einer Informationsübertragung zwischen mehreren Kommunikationsmitteln durch einen menschlichen Körper (101), umfassend:
ein erstes Kommunikationsmittel (102), das eine erste Elektrode (104) aufweist, die so angeordnet ist, dass sie zu dem menschlichen Körper (101) weist und sich in direktem Kontakt mit dem menschlichen Körper befindet oder kapazitiv an diesen angeschlossen ist, sowie eine zweite Elektrode (105), und das verwendet wird, während es an dem menschlichen Körper befestigt ist, und
ein zweites Kommunikationsmittel (106), das eine dritte Elektrode (108) aufweist, die so angeordnet ist, dass sie zu dem menschlichen Körper (101) weist und sich in direktem Kontakt mit dem menschlichen Körper befindet oder kapazitiv an diesen angeschlossen ist, sowie eine vierte Elektrode (109), die so angeordnet ist, dass sie von dem menschlichen Körper weg weist, und das verwendet wird, während es an dem menschlichen Körper befestigt ist,
wobei das Informationsübertragungssystem dazu ausgebildet ist, eine Kommunikation zwischen dem ersten und zweiten Kommunikationsmittel (102, 106) durch eine elektrische Verbindung zwischen der ersten Elektrode (104) und der dritten Elektrode (108) durch den menschlichen Körper und durch eine kapazitive Verbindung zwischen der zweiten Elektrode (105) und der vierten Elektrode (109) auszuführen;
**dadurch gekennzeichnet, dass**:
die zweite Elektrode (105) so angeordnet ist, dass sie zu dem menschlichen Körper (101) weist, und sich in direktem Kontakt mit dem menschlichen Körper befindet oder kapazitiv an diesen angeschlossen ist;
die dritte und vierte Elektrode (108, 109) durch einen Hauptkörper (107) voneinander getrennt sind, wobei die dritte und vierte Elektrode (108, 109) an gegenüberliegenden Enden des Hauptkörpers (107) angeordnet sind; und
die kapazitive Verbindung zwischen der zweiten und vierten Elektrode (105, 109) über den menschlichen Körper (101) erfolgt.

2. Informationsübertragungssystem über den menschlichen Körper nach Anspruch 1, **dadurch gekennzeichnet, dass** das Informationsübertragungssystem zur Übertragung einer Organismusinformation des menschlichen Körpers (101) zwischen dem ersten Kommunikationsmittel (102) und dem zweiten Kommunikationsmittel (106) ausgebildet ist, und zur Verwendung der ersten und zweiten Elektrode (104, 105) des ersten Kommunikationsmittels sowohl für eine Elektrode, die die Organismusinformation des menschlichen Körpers erfasst, wie auch für eine Elektrode, die eine Übertragung der Organismusinformation durchführt.

3. Informationsübertragungssystem über den menschlichen Körper nach Anspruch 1 oder 2, **dadurch gekennzeichnet, dass** das Informationsübertragungssystem ausgebildet ist zum Einstellen einer Frequenz eines Trägersignals, das in der Kommunikation zwischen dem ersten Kommunikationsmittel (102) und dem zweiten Kommunikationsmittel (106) verwendet wird, auf eine Frequenz, die sich von einer Frequenz der Organismusinformation des menschlichen Körpers (101) unterscheidet.

4. Informationsübertragungssystem über den menschlichen Körper nach Anspruch 3, **dadurch gekennzeichnet, dass** die Frequenz des Trägersignals eine Frequenz innerhalb eines Bereichs von 5 MHz - 45 MHz ist.

5. Informationsübertragungssystem über den menschlichen Körper nach einem der Ansprüche 2 bis 4, **dadurch gekennzeichnet, dass** die Organismusinformation eine Information über einen Herzschlag ist.

6. Informationsübertragungssystem über den menschlichen Körper nach einem der Ansprüche 1 bis 5, **dadurch gekennzeichnet, dass** das erste Kommunikationsmittel (102) an dem menschlichen Körper (101) durch ein Brustband (110) befestigt ist.

7. Informationsübertragungssystem über den menschlichen Körper nach einem der Ansprüche 1 bis 6, **dadurch gekennzeichnet, dass** das erste Kommunikationsmittel (102) ein Filter (202) aufweist, das zum Weiterleiten und Ausgeben eines Signals einer vorbestimmten Frequenz in einem Signal ausgebildet ist, das von der ersten Elektrode (104) empfangen wurde, sowie einen Herzschlagerfassungsabschnitt (203), der zum Erfassen eines Herzschlagsignals von einem Ausgangssignal des Filters ausgebildet ist, einen ersten Prozessorabschnitt (204), der zum Berechnen einer Herzschlagzahl auf der Basis des Herzschlagsignals ausgebildet ist, das in dem Herzschlagerfassungsabschnitt erfasst wurde, um **dadurch** ein Herzschlagzahldatensignal auszugeben, das die Herzschlagzahl angibt, einen Modulationsabschnitt (205), der zum Modulieren und Ausgeben des Herzschlagzahldatensignals ausgebildet ist, und einen Datenübertragungsabschnitt (206), der zum Ausgeben des Herzschlagzahldatensignals, das in dem Modulationsabschnitt moduliert wurde, ausgebildet ist, und
das zweite Kommunikationsmittel (106) einen Demodulationsabschnitt (302) aufweist, der zum Demodulieren des Herzschlagzahldatensignals ausgebildet ist, das von der dritten Elektrode (108) empfangen wurde, sowie ein Speichermittel (304), einen Anzeigeabschnitt (305) und einen zweiten Prozessorabschnitt (303), der zum Speichern von Herzschlagzahldaten in dem Speichermittel (304) ausgebildet ist, die von dem Herzschlagzahldatensignal erhalten wurden, das in dem Demodulationsabschnitt moduliert wurde, und zum Verarbeiten der Herzschlagzahl zur Anzeige im Anzeigeabschnitt (305).

## Revendications

1. Système de transmission d'informations via le corps humain pour effectuer une transmission d'informations entre des moyens de communication multiples par l'intermédiaire d'un corps humain (101), comprenant :
un premier moyen de communication (102) ayant une première électrode (104) disposée de manière à faire face à un corps humain (101) et qui est directement en contact avec le corps humain ou est couplée de manière capacitive à celui-ci, et une deuxième électrode (105), et qui est utilisé pendant qu'il est fixé sur le corps humain, et
un deuxième moyen de communication (106) ayant une troisième électrode (108) disposée de manière à faire face au corps humain (101) et qui est directement en contact avec le corps humain ou est couplée de manière capacitive à celui-ci, et une quatrième électrode (109) disposée de manière à tourner le dos au corps humain, et qui est utilisé pendant qu'il est fixé sur le corps humain ;
dans lequel le système de transmission d'informations est adapté pour effectuer une communication entre le premier et le deuxième moyen de communication (102, 106) grâce à une connexion électrique entre la première électrode (104) et la troisième électrode (108) par l'intermédiaire du corps humain, et grâce à une connexion capacitive entre la deuxième électrode (105) et la quatrième électrode (109) ;
**caractérisé en ce que** :
la deuxième électrode (105) est disposée de manière à faire face au corps humain (101) et se trouve en contact direct avec le corps humain ou est connectée de manière capacitive à celui-ci;
les troisième et quatrième électrodes (108, 109) étant séparées l'une de l'autre à l'aide d'un corps principal (107), les troisième et quatrième électrodes (108, 109) étant disposées sur des extrémités opposées du corps principal (107) ; et la connexion capacitive entre les deuxième et quatrième électrodes (105, 109) ayant lieu à l'aide du corps humain (101).

2. Système de transmission d'informations via le corps humain selon la revendication 1, **caractérisé en ce que** le système de transmission d'informations est adapté pour transmettre une information d'organisme du corps humain (101) entre le premier moyen de communication (102) et le deuxième moyen de communication (106), et pour utiliser les première et deuxième électrodes (104, 105) du premier moyen de communication à la fois en tant qu'électrode détectant l'information d'organisme du corps humain et d'électrode effectuant une transmission de l'information d'organisme.

3. Système de transmission d'informations via le corps humain selon la revendication 1 ou 2, **caractérisé en ce que** le système de transmission d'informations est adapté pour fixer une fréquence d'un signal de porteuse utilisé dans la communication entre le premier moyen de communication (102) et le deuxième moyen de communication (106) à une fréquence différente d'une fréquence de l'information d'organisme du corps humain (101).

4. Système de transmission d'informations via le corps humain selon la revendication 3, **caractérisé en ce que** la fréquence du signal de porteuse est une fréquence dans une plage de 5 MHz - 45 MHz.

5. Système de transmission d'informations via le corps humain selon l'une quelconque des revendications 2 à 4, **caractérisé en ce que** l'information d'organisme est une information sur un battement du coeur.

6. Système de transmission d'informations via le corps humain selon l'une quelconque des revendications 1 à 5, **caractérisé en ce que** le premier moyen de communication (102) est fixé sur le corps humain (101) à l'aide d'une bande pour poitrine (110).

7. Système de transmission d'informations via le corps humain selon l'une quelconque des revendications 1 à 6, **caractérisé en ce que** le premier moyen de communication (102) possède un filtre (202) adapté pour faire passer et sortir un signal d'une fréquence prédéterminée dans un signal ayant été reçu par la première électrode (104), une section de détection de battement du coeur (203) adaptée pour détecter un signal de battement du coeur d'un signal de sortie du filtre, une première section de processeur (204) adaptée pour calculer un nombre de battements du coeur sur la base du signal de battement du coeur ayant été détecté dans la section de détection de battement du coeur afin de faire sortir ainsi un signal de données du nombre de battements du coeur indiquant le nombre de battements du coeur, une section de modulation (205) adaptée pour moduler et faire sortir le signal de données du nombre de battements du coeur, et une section de transmission de données (206) adaptée pour faire sortir le signal de données du nombre de battements du coeur ayant été modulé dans la section de modulation, et
le deuxième moyen de communication (106) ayant une section de démodulation (302) adaptée pour démoduler le signal de données du nombre de battements du coeur ayant été reçu par la troisième électrode (108), un moyen de stockage (304), une section d'affichage (305), et une deuxième section de processeur (303) adaptée pour stocker dans le moyen de stockage (304) des données sur le nombre de battements du coeur ayant été obtenues de la part du signal de données du nombre de battements du coeur ayant été démodulé dans la section de démodulation, et pour afficher-traiter le nombre de battements du coeur sur la section d'affichage (305).
